# EUROPEAN PATENT APPLICATION

(11) **EP 1 450 159 A2**
(43) Date of publication of application: **25.08.2004**
(21) Application number: 04250998.4
(22) Date of filing: 24.02.2004
(51) Int. Cl.: G01N 33/49, B01L 3/14

(54) **Method and apparatus for the detection of agglutination of assays**

(30) Priority: 24.02.2003 US 372745
(71) Applicant: ORTHO-CLINICAL DIAGNOSTICS, INC., Rochester, New York 14626-5101 (US)
(72) Inventor: Moulds, John, Pipersville, PA 19847 (US); Zislin, Alex M., Princeton Junction, New Jersey 08550 (US); Szucs, John, Morris Plains, New Jersey 07950 (US)
(74) Representative: Mercer, Christopher Paul

(57) **Abstract**

An apparatus for conducting an assay having an agglutination or size separation step, includes: a first section disposed to receive a fluid to be assayed; and a second section disposed to receive the fluid from the first section upon application of a motive force, preferably centrifugal force, to the fluid, the second section comprising elements fixed to a substrate and adapted to mix the fluid and trap agglutinated particles. In a preferred embodiment, the elements are shaped as pillars. In another preferred embodiment, a third section is provided after the second section and the apparatus is in the form of a disk, preferably an optical disk, having a central axis, and wherein the first, second and third section are arranged in the disk as channels in a direction away from the central axis, respectively. A method for assaying a fluid that has particles to be separated or agglutinated includes: providing a fluid to be assayed into a first section of an apparatus as described above; applying a motive force to the fluid to move the fluid from the first section into a second section, wherein said second section comprises elements fixed to a substrate and adapted to mix the fluid and trap particles in the fluid; and measuring a property of the fluid. In a preferred embodiment, the fluid to be assayed is blood.

## Description

### BACKGROUND OF THE INVENTION

### Field of the Invention

This invention relates to the field of agglutination assays, and particularly to an apparatus useful for conducting assays having an agglutination or size separation step, and for separating agglutinates, particularly red blood cells.

### Background of the Invention

Blood group serology requires the determination of blood cell compatibility between a blood donor and patient recipient before a transfusion or organ transplant involving the patient. Blood cell compatibility is determined by the absence of immunological reaction between antibodies contained in the blood serum of a patient and antigens present on blood cells from the donor. Many different blood group antigens are found on the surface of red blood cells of every individual. Blood grouping is generally the process of testing red cells to determine which antigens are present and which are absent. This is generally accomplished by using antibodies of known specificity.

For detecting antibodies in the serum or plasma of a patient, reagents containing blood cells having known antigens are mixed with a serum sample. The reactants are'incubated for a period of time sufficient to permit agglutination of the red blood cells, which occurs when antibodies against those antigens are present. The mixture is then centrifuged, and if agglutinated blood cells are present, such agglutinates are clearly visible at the bottom of the reaction vessel, thus indicating the presence of antibodies in the sample directed against the known antigens on the red blood cells. If no antibodies are present in the sample directed against the known antigens on the red blood cells, agglutination does not occur, and this is indicated by the absence of agglutinated red cells after centrifugation.

More recently, systems have been developed in which the agglutination reaction is carried out in one portion of a vessel, and separation of agglutinated red cells is accomplished in another portion of the same vessel using a matrix which separates agglutinated cells from other components in the reagent/sample mixture. One such system is disclosed and described in U.S. Patent Nos. 5,650,068 and 5,552,064, both of which are commonly owned by the owner of the subject application. The contents of each of these patents are hereby incorporated by reference herein. Such agglutination reaction and separation vessels, and which are also useful in the inventions disclosed in the aforementioned applications, are manufactured and sold by Ortho-Clinical Diagnostics Inc., Raritan, New Jersey, under the trademark BIOVUE®. Such reaction vessels are in the form of a column having an upper chamber and a lower chamber wherein the upper chamber is of a wider diameter than the lower chamber. The lower chamber contains a matrix for separating agglutinated cells from non-agglutinated cells. The diameter of the lower chamber is narrow enough such that when reagents and samples are added to the upper chamber, typically using a pipette, the reagents and samples remain in the upper chamber, and do not enter into the lower chamber, unless an additional force is applied.

An indirect antiglobulin test, known as the Coombs test, is a blood test used to determine whether there are IgG antibodies in a patient's serum to specified antigens on the surface of red blood cells. In the Coombs test, serum is incubated in the presence of reagent red cells to allow the antibodies to bind to antigens on the surface of the red cells. These IgG antibodies most often do not, by themselves, agglutinate the red cells, or only agglutinate them insufficiently to be detected visually by conventional techniques. Addition of a second antibody directed to human IgG is usually necessary to facilitate visible agglutination.

In red cell typing, a blood test used to determine whether certain antigens are present on the surface of red blood cells, the red cells being analyzed are added to the upper chamber followed by application of centrifugal force which moves them into the lower chamber containing antibodies to particular red cell antigens and the separation matrix. If the red cells have the antigen(s) on their surface to combine with the specific antibodies in the lower chamber, agglutinates will form and be separated by the matrix.

In other types of blood assays, such as reverse typing where directly agglutinating antibodies for red cell antigens in a patient's serum are being assayed, a patient's serum and reagent red blood cells, with known antigens on their surface, are added to the upper chamber and centrifugal force is applied to move the reactants into a lower chamber which contains a liquid medium and separation matrix but no antibody. In this assay the presence of directly agglutinating antibody in the patient's serum would produce agglutinates which would be separated by the matrix.

In another type of blood assay, reagent antibody with a known specificity for a red cell antigen would be deposited into the upper chamber, together with patient's red cells. If the reagent antibody is a directly agglutinating antibody, centrifugal force would be applied without prior incubation and the contents would be forced into the lower chamber containing separation matrix in aqueous solution. Agglutinates would then be separated by the matrix. Alternatively, patient's red cells are deposited into the upper chamber and IgG reagent antibody with known specificity is added, followed by incubation to allow the antibody to attach to presumptive antigens on the surface of the red cells. After incubation, centrifugal force is applied to move the reactants into the lower chamber which contains separation matrix and anti-IgG antibodies specific for the IgG reagent antibody used to incubate reed cells in the upper chamber. If the reagent antibody is present on the surface of the patient's cells, the anti-IgG antibody in the lower chamber would facilitate the formation of agglutinates which would be separated by the matrix.

After the sample and reagents have been allowed to incubate for a sufficient period of time to permit either direct agglutination, as in the case of a red cell typing test, an antibody-antigen reaction, as in the case of a Coombs test, the reaction vessel is centrifuged so that the reactants are expelled into the lower portion of the column and onto the separation matrix. As a result of the centrifugation, unagglutinated materials migrate down through the separation matrix while agglutinated cells remain on top of the separation matrix or distributed within the matrix depending on the degree of agglutination. Stronger agglutination reactions result in the cells remaining towards the upper portion of the separation matrix while weaker agglutination reactions result in distribution of agglutinates at various distances from the top of the matrix.

Retention of the sample and reagents in the upper portion of the column during the incubation phase is the result of surface tension across the top margin of the lower portion of the column where the diameter is reduced relative to the upper portion. Two potential sources of error in conducting an assay using this column have been identified. First, if reagents and sample are pipetted directly down the center of the reaction chamber with excessive force, the reactants may be deposited directly to the top of the separation matrix in the lower chamber and not retained in the upper chamber during the incubation phase. Thus, the reactants will begin to enter the separation matrix prior to the completion of agglutination. Second, there is potential that the diluent or solution which contains the separation matrix may enter the upper chamber. This can occur through splashing or other disturbance, for example, during shipping and handling of the vessels. In some cases where the solution or diluent containing the separation matrix also contains antibodies or other reagents which directly affect the result of a test, such splashing can result in cross-contamination of columns with certain reagents from other columns. This may occur when the user inserts a pipette tip into the reaction chamber, contaminating the tip with splashed reagent, which may then be transferred to another vessel by the pipette. This may lead to false results in the agglutination assay.

The aforementioned traditional methods for determining red cell blood grouping (testing for red cell membrane protein polymorphic variations) has been performed by mixing red cells (either as whole blood or in a suspension of cells in a physiological suspension fluid) with a fluid containing either human or animal antibodies or lectins. As discussed hereinabove, the endpoint of a positive reaction is the detection of agglutination due to the clumping of red cells in the presence of specific antibodies to red cell membrane structure. Red cells that do not so agglutinate are considered to be negative or lacking in the specific, tested membrane structure, or blood group. Such tests have been performed on glass slides or within test tubes, most commonly in 10x75mm or 12x75mm test tubes. To accelerate the juxtaposing of the red cells and thus the rate of reaction the test when performed in the test tube may be centrifuged and the thusly sedimented red cells suspended by gently shaking the tube. Traditionally, when agglutination was detected within a fluid medium, interpretation of the results may be subjective and is considered a procedure that requires a highly skilled and knowledgeable operator.

Recently methods have been described whereby the fluid mixture of antibody and red cells are mixed and allowed to incubate for a period of time prior to the mixture being sieved through a porous matrix which allows agglutinated cells to be separated from non-agglutinated cells. Generally those cells that are agglutinated or clumped will not sieve through the small pores and will be trapped on the surface of the porous material whereas the non-agglutinated cells will filter through the porous material. This method of testing requires minimal special training to discern agglutinated from non-agglutinated cells and can easily be read by automated devices. The sieving material can be made by a variety of particles such as small spherical gel or glass beads, glass wool or fibers, etc., the most popular being Sephacryl^{TM} (Amersham Pharmacia Biotech AB) or glass beads. Descriptions of such materials and methods are described in U.S. Patent Nos. 5,460,940 and 5,491,067 to La Pierre et al., both of which are incorporated by reference in their entireties.

As discussed hereinabove, the sieving materials are intended to separate agglutinated from non-agglutinated red cells. The general principle is the sieving effect administered to the red cell suspension traveling under gravitational or centrifugal force through the spatial-void or passageways between the spheres of porous material (the glass, Sephycryl^{TM}, or other material appropriate to the chemistry of the assay). The size of these passages are determined by the size of the solid spherical particles. Practice has shown that Sephycryl^{TM}, having an average bead size of 50 microns, which thus spans a particle range size of 25-75 microns, forms suitable sized passageways between the spheres. Passageways of this size are incapable of trapping unagglutinated human red cells but capable of trapping agglutinated cells of various sizes that are critical to the serological performance criteria of blood grouping. Similar results have been observed when using glass beads of an average size of 70-80 microns in diameter. Passageways thus formed are determined to be on the order of 6-15 microns in width depending on orientation and packing density.

The commonality of the two most popular blood grouping methods using the principles of agglutination detection is that the sieving mechanism, for example, the Sephycryl^{TM} or glass beads are independent objects from the testing vessel, such vessel frequently referred to as the tube/microtube or column/microcolumn. These sieving agents are mixed as solid particles within the formulation solution to be inserted into the testing vessel or are placed within the testing vessel either prior to or after the liquid formulations are added. The necessity of maintaining the solidity of shape to ensure proper passage size is an important consideration.

Virtanen (U. S. Patent No. 6,030,581) discloses an optical disk format for performing blood analyte testing. Depending on the nature of the assay the disclosed disk includes the many elements of a fluid storage means, fluid transfer means, such as one or more capillary ducts, valves, batteries, dialyzers, columns, filters, sources of electric fields, wires or other electrical conductive means such as metallic surface deposits and the like.

WO 97/21090 discloses a device that uses centripetal action to drive fluid movement in a microfluidics system with on-board informatics.

### SUMMARY OF THE INVENTION

One object of the invention is to overcome the disadvantages of the known art as described above. Another object of the invention is to avoid the aforementioned potential errors connected with the practice of tube and chamber agglutination testing. Another object of the present invention to provide an improved method and device for carrying out an assay by agglutination, particularly for blood typing. It is a further object of the invention to provide a system, particularly an automated system, that is capable of performing an agglutinating assay with increased speed and accuracy.

The foregoing and further objects of the invention are accomplished according to one aspect of the invention, which provides an apparatus for conducting an assay having an agglutination or size separation step, which includes: a first section disposed to receive a fluid to be assayed; and a second section disposed to receive the fluid from the first section upon application of a motive force to the fluid, the second section including elements fixed to a substrate and adapted to mix the fluid and trap agglutinated particles. In a preferred embodiment, the elements are shaped as pillars. In another preferred embodiment, a third section is provided after the second section and the apparatus is in the form of a disk, preferably an optical disk having a central axis, and wherein the first, second and third section are arranged in the disk as channels in a direction away from the central axis, respectively.

Another aspect of the invention, provides an apparatus for conducting an assay having an agglutination or size separation step. The apparatus includes: an optical disk having a central axis; a first section disposed to receive a fluid to be assayed, said first section including a fluid entry port to provide the fluid to be assayed; a second section disposed to receive the fluid from the first section upon application of a motive force to the fluid, the second section including elements fixed to a substrate and adapted to mix the fluid and trap agglutinated particles; a third section disposed to receive fluid from the second section, wherein the first, second and third section are arranged in the disk as channels in a direction away from the central axis, respectively; a drive for rotating the apparatus around the central axis; and an optical detector.

Another aspect of the invention provides a method for assaying a fluid that has particles to be separated or is agglutinated. The method includes: providing a fluid to be assayed into a first section of an apparatus as described above; applying a motive force to the fluid to move the fluid from the first section into a second section, wherein the second section includes elements fixed to a substrate and adapted to mix the fluid and trap particles in the fluid; and measuring a property of the fluid. In a preferred embodiment, the fluid to be assayed is blood. Another aspect of the invention provides a method for agglutinating blood, which includes: providing blood or into a first chamber; providing a reagent; combining the reagent and the blood; applying a motive force to move the combined reagent and blood from the first section into a second chamber. The second chamber includes elements fixed to a substrate and adapted to mix the blood and reagent and trap agglutinated blood cells.

Another aspect of the invention provides an article of manufacture that includes a computer usable medium having computer readable program code configured to conduct the methods described above.

Further objects, features and advantages of the present invention will be apparent to those skilled in the art from detailed consideration of the preferred embodiments that follow.

### BRIEF DESCRIPTION OF THE DRAWINGS

Figure 1 shows a sectional top view of the apparatus according to one aspect of the present invention.
Figure 2 shows a sectional top view of the apparatus according to another aspect of the present invention.
Figure 3 shows a sectional top view of the apparatus, including the carrier, according to another aspect of the present invention.
Figure 4 shows a sectional top view of the apparatus, including the carrier, according to another aspect of the present invention.
Figure 5a shows a partial, schematic sectional side view of the apparatus according to another aspect of the present invention.
Figure 5b shows a partial sectional top view of the apparatus illustrating the separating elements according to the embodiment shown in Figure 5a.
Figure 6 shows a sectional top view of the apparatus, including the carrier, according to another aspect of the present invention.
Figure 7 shows a schematic sectional top view of the apparatus, including the carrier, according to another aspect of the present invention.
Figure 8 shows a schematic sectional top view of the apparatus, including the carrier, according to another aspect of the present invention.
Figure 9 shows a perspective view of another embodiment of the apparatus, including the carrier, according to the present invention.
Figure 10 shows a perspective view of another embodiment of the apparatus, including the carrier, according to the present invention.
Figure 11 shows a system that includes the apparatus according to the present invention.
Figure 12a is a top view of the apparatus, including the carrier, where the apparatus is designed as an insert.
Figure 12b is a sectional side view of the apparatus according to Figure 12a.

### DETAILED DESCRIPTION OF PREFERRED EMBODIMENTS

As described in the background section, known column agglutination technology (CAT) platform uses beads (e.g., BioVue^{TM}) or gel (e.g., DiaMed^{TM}/MTS^{TM}) as a device to separate different sizes of agglutination. Each agglutination level/strength (i.e. 0, +1, +2, +3, and +4) migrates different distances down the column under centrifugation. The agglutination band is then detected and graded either visually by a blood bank technician, or automatically by an instrument such as the AutoVue^{TM}. The present invention uses column agglutination technology (CAT) for blood typing of human red cells as an exemplary embodiment in describing the present invention; however, the present invention described in this disclosure is not limited to human red cells, but rather can be applied to any particulate analyte that requires agglutination or size separation as an assay measurement, such as measuring latex particles in a solution.

As used herein, "blood" broadly includes whole blood or any component of whole blood, such as red blood cells, plasma, serum, etc.

The apparatus of the present invention includes a first section for receiving a fluid to be assayed, and a second section that receives the fluid to be assayed from the first section upon application of a motive force to the fluid. The first section can be any structure, such as a chamber, capable of receiving and holding a fluid, such as serum or plasma to be typed and the corresponding reagents. In a preferred embodiment, the apparatus is formed in a disk, such as an optical disk, e.g., a compact disk (e.g., CD-ROM) configuration and all three sections are formed as a channels in the disk. However, other configurations, such as glass or plastic slides, or variation of these technologies could also be used. In a preferred embodiment, the first section is separated into two sections, such as a sample receiving section and a reactant receiving section. A baffle or plate may be interposed between the two sections to prevent the fluids from commingling until the application of a motive force. Upon application of the motive force, the fluids are forced over and around the baffle or plate into intimate contact with each other. Alternatively, the first section may have only a single chamber. In this instance, mixing would occur by the agitation and intermingling of fluids when they are added to the first section, such as through an opening in the cover of the first section.

The second section of the apparatus can be similar to the first section. The second section may be sized to take advantage of surface tension. That is, the second section (or entrance to the second section) may be sized such that the fluid will not enter by gravity or capillary action alone without the application of a motive force such as centripetal force.

An important feature located in the second section are the elements fixed to a substrate. The elements may take the place of the beads or gel that is conventionally used in CAT devices. The manufacturing issues of bead formation, sizing and finishing and manufacturing defects are greatly lessened, or preferably eliminated, as the present invention does not make use of beads, bead-like particles or gels. The problem of filling CAT cassettes with either beads or gels is also eliminated, and the problems of shipping environments causing the formation of bubbles is also eliminated or greatly reduced.

The only requirement of the shape of the elements and the distance between the elements is that they are able to function to separate the agglutinated particles, such as agglutinated red blood cells. The shape, for example, may be cylindrical pillars attached to and arising from the base surface or substrate. The elements may be organized in rows so the positioning of the pillars in the rows are offset such that as the fluid flows through the array of pillars it does not allow flow of red cells and agglutinates in a straight passage between the pillars in adjacent rows. The distance between the pillars may be further apart in one area so as to trap only large agglutinates, and the distance may, for example, be gradually decreased downstream so as to selectively separate agglutinates of varying sizes. Other appropriate shapes for the elements may include triangular-shaped, diamond-shaped, or conical-shaped pillars.

The elements are fixed to a substrate. The substrate may be the chamber walls of the second section, or may be a pre-formed insert that is inserted into the second section at some point before use. The elements can be formed to the substrate in one-piece, such as by injection molding. Alternative methods can also include machining or etching the elements from a substrate. By using an appropriate process it is possible to form elements and channels of a size appropriate for separating agglutinates into a particular pattern. For example, in the case of agglutinated red blood cells, elements may extend from a flat surface base with a distance of not less than ten (10) microns between them. In a preferred embodiment where red blood cells are being agglutinated, the elements have a diameter of 40 to 80 microns and the distance between the elements is 7-15 microns. An additional advantage of the present invention is that the agglutination reaction is enhanced or amplified thus making the detection of weak positives more reliable, in that the reaction section and separation section allows for a more complete reaction, thus enhancing/amplifying the reaction of weak positives.

In a preferred embodiment, a valve is located between the first and second zone. A preferred function of the valve is to transition the fluid from the first section to the next section and to promote mixing during the transition. Another possible function of the valve is to hold the fluid in the first section until a predesired condition is reached, such as the application of a predetermined force. Preferably the valve is a static valve. The static valve can be any sufficient structure to achieve this function. For example, in a preferred embodiment, the static valve is a series of steps or ridges.

In a preferred embodiment, an enhancement section or zone is located between the first and second sections. This is a zone that provides additional mixing of the fluid containing particles, such as the red blood cells in the process of agglutinating. This causes the fluid to increase its turbulence and causes any particles to increase their proximity to enhance the strength of a reaction, such as an agglutination reaction. The enhancement section can include protrusions or other structures to interfere with the flow of fluid through the section. For example, the enhancement section can include baffles.

In a preferred embodiment, the invention also includes a third section located downstream from the second section for receiving fluid and or agglutinates from the second section.

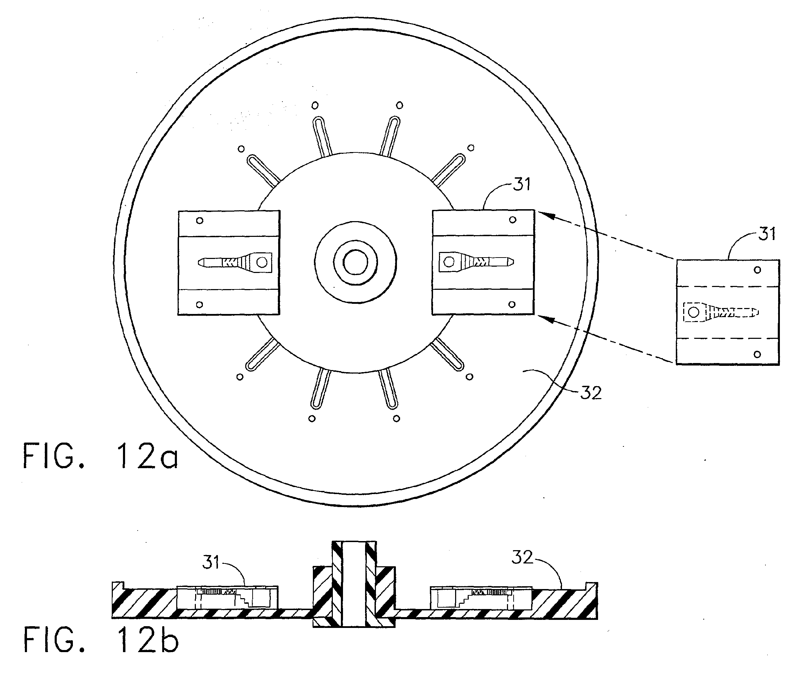

In another preferred embodiment, the invention can also include a housing for containing the sections of the apparatus. In some embodiments, the housing itself forms the chambers for the sections. That is, the housing and chambers are a one-piece construction. In other embodiments, the section chambers can be a structure separate from the housing, such as inserts as illustrated in Figures 12a and 12b. In a preferred embodiment, the housing is a disk having a central axis, preferably an optical disk if optical detection is used, such as a CD-ROM device described more fully below. In another embodiment, the housing can be a slide.

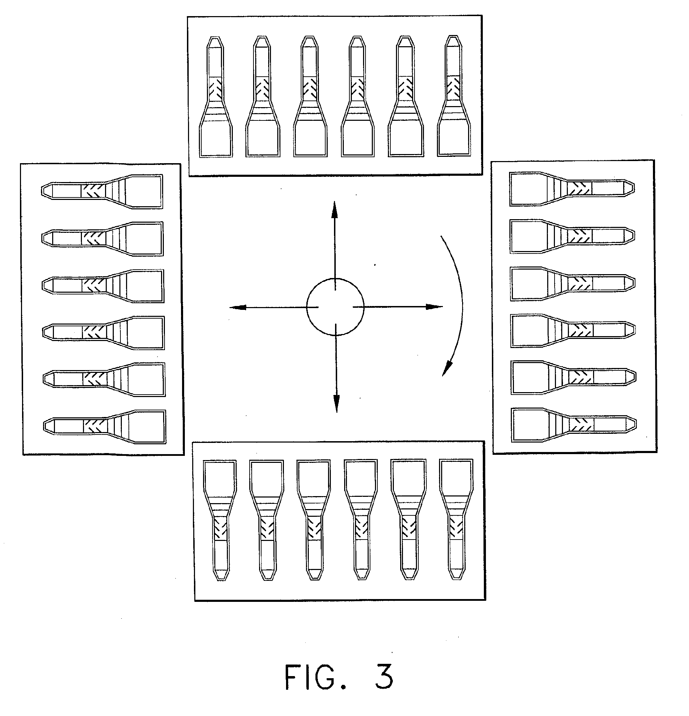

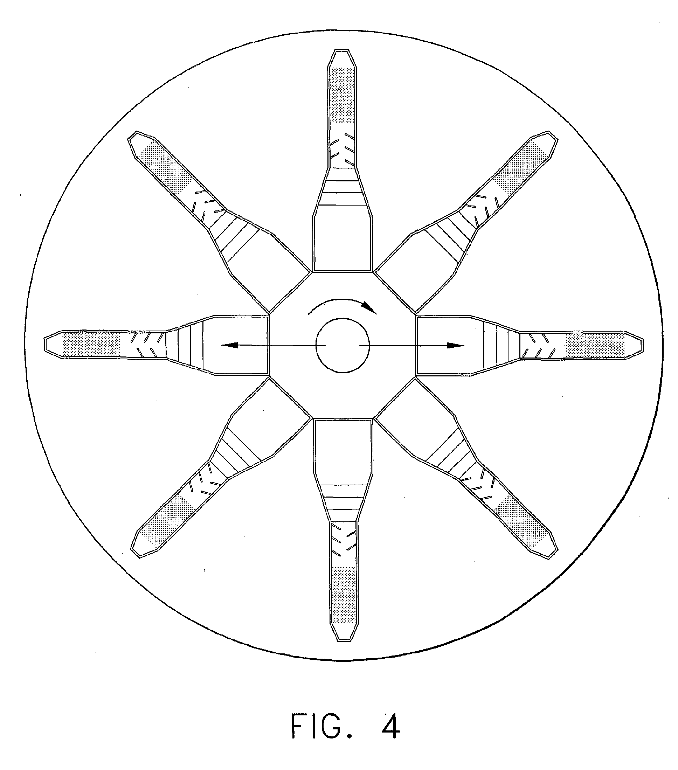

In another aspect of the invention, the apparatus includes one or more of a first, second and third sections, preferably a plurality, and a carrier for holding the plurality of sections. The carrier can arrange the plurality of sections in any suitable manner as long as motive force can be applied to move the fluid from the first section through the second section. The arrangement can be side-by-side, or preferably arranged around a central axis as shown in Figure 4. In a preferred embodiment, the carrier holds the sections in a side-by-side configuration, and multiple carriers are arranged around a central axis as shown in Figure 3. Where the sections and/or carriers are arranged around a central axis, the motive force can be conveniently provided by centripetal force generated by rotating the carrier(s). In a preferred embodiment, the carrier is in a known CD format such as described in U.S. Patent No. 6,030,581, incorporated herein by reference in its entirety.

Detecting the presence of agglutination and/or the extent of agglutination can be carried out using detection schemes well known in the art. For example, detection could be similar to the current detection systems used on known instruments, such as the AutoVue® instrument. In another detection method, the agglutination complex moves through the separation area at a rate dependent upon the g-forces (or other motive force being applied), the size of the agglutination, and the pore or opening size in the separation area. By imaging the device multiple times during centrifugation, this rate may be determined by processing the image and locating the agglutination complex in relation to the starting point and length of the separation area. In addition, the final position is also determined.

If optical imaging is employed, it may be performed either by a 'staring' system, or by a scanning system. The difference is in the configuration of the imaging optics and the pre-processing of the image pixels to assemble the image. Pixel resolutions and sample rates are determined to meet requirements of minimum image clarity and feature edge determination.

Other detection methods may also be used. For example, the assay may be configured such that the agglutination complex includes a marker such as iron particles, fluorescent compounds, chromgenic compounds (i.e. OPD or TMB), or radioactive tracers. Detection may then be performed via methods such as magnetic detection, capacitive measurements, optical density measurements, optical imaging, spectrophotometric, or radiation measurements. The structure and design of the present invention allows for these alternatives.

In one preferred detection method, the apparatus is a disk made of an optically clear material and the particles have some degree of color such that the results can be read visually, with or without optical aids. In this preferred method, the detection would occur after the disk is no longer in motion. The disk could be read while still on the device that provides motion, e.g., a rotor, or removed and read over a lighted background.

In another embodiment, an automated reading can be used, such as the optical imaging described above. With automated reading, the results can be determined while the disk is still on the rotor. In one embodiment, an optical detection device is located either above or below the disk, and its view of the relevant areas of the disk (i.e., the second and third sections) is through a slit running the length of the viewed area. The viewed area is illuminated from the opposite side of the disk. The detector distinguishes the degree of light or color transmittance simultaneously through the entire length of the slit. This reading is then subdivided into small quadrants in such a manner that the optics system is able to compare light transmission or light interference from one area of the slit to another. A summation of the optical analysis is then generated with a logic program to distinguish positive from negative results. An advantage of automated reading is that it is possible to analyze the results while the disk is in motion. Thus, the slit could be viewed through the entire application of motive force, such as centrifugation. This allows multiple calculations to be made on any test by comparing the flow rate and placement of the agglutinated/non-agglutinated mass at a specific location in the slit area during a controlled centrifuge time and/or centrifuge speed. These could produce the final results to be determined, thus abolishing any stationary reading. Alternatively, the moving and stationary results could be combined for final results.

The motive force can be any force capable of moving the fluid through the apparatus and can include an electric field, a magnetic field, a hydrodynamic force, a hydrostatic force, a gravitational force, a centrifugal force, an optical force and a thermal force. Preferably the force is centrifugal force.

The apparatus of the invention may be used in a system for determining agglutination of a plurality of samples. For example, the system may include the apparatus and multiple carriers, such as CD's. A carrier transport can also be included to transport the carriers to sample and/or reagent fill. A sample and reagent pipetting and positioning station can also be included to load the apparatus with sample and reagent. The system may further include an incubator, centrifuge for applying motive force to the fluid, a detector and reader. Associated control components such as controllers, computer terminals and data input drivers may also be included.

The present invention also provides a method for assaying a fluid, such as blood. Broadly, any fluid that has particles, such as red blood cells or latex particles, to be separated or agglutinated can be assayed according to the present invention. In one embodiment of the invention, the fluid is provided into the first section of the apparatus described above, or simply into a first chamber. If required, a reagent, such as an antibody can also be supplied. A motive force is then applied to the fluid to move the fluid from the first section into a second section of the chamber described above, or simply a second chamber. The second chamber has elements fixed to a portion of the second chamber to mix the fluid and trap particles in the fluid. The motive force is preferably applied by spinning the second section or chamber as described above, resulting in the application of centrifugal force. The apparatus containing the second section or second chamber is preferably an optically transparent rotatable disk. After the fluid has moved through the second section or chamber, separation of the particles present in the fluid, if any, will have occurred and measuring a property of the fluid, such as the degree of separation in the case of blood agglutination can be performed. The measurement can be any required for the particular assay being performed.

Many of the assays that can be performed on blood include, ABO grouping, Rh typing, antigen phenotyping, ABO serum grouping, antibody detection and identification, crossmatching and titration.

In a preferred embodiment, the methods described above can be implemented by a computer program interfacing with a computer, that can include a computer usable medium having computer readable program code configured to conduct the methods.

The present invention can also broadly be used to simply agglutinate or separate a fluid using the method described above, but without necessarily performing the final measurement on the separated fluid.

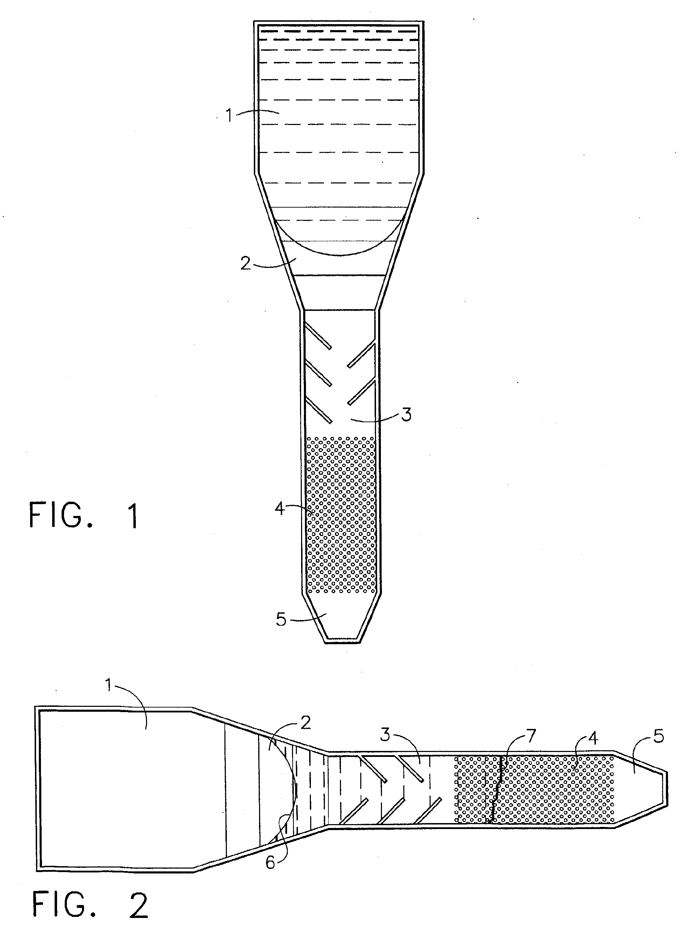

Now reference will be made to the detailed description of preferred embodiments shown in the figures. The embodiment shown in Figure 1 includes a chamber (1) for the initial reaction between the analyte and the reagents. At the end of the incubation period, the device is spun on a centrifuge such that the liquid containing the reagents and analyte, such as plasma, passes over a static valve (2) which serves to keep the liquid out of the separation and detection area until required. The static valve contains the liquid in the reaction chamber (1) via, for example, surface tension, or physical baffling. Centrifugation of the device overcomes the restraining mechanism and allows the analyte and reagent to enter the enhancement (3) and separation (4) sections. The enhancement section (3) serves to cause the particles to increase their proximity thus enhancing the strength of the agglutination reaction. The separation section (4) consists of fixed barriers or elements with defined opening sizes such that the agglutination complex is separated by size. The size of the openings may be variable or constant depending upon the design of the assay, and the expected size and distribution of the agglutination complex. The shape of the barriers may be cylindrical, conical, diamond or rectangles, or any other shapes that function to separate the agglutinates by size.

As described above, the detection in the embodiment shown in Figure 1 is similar in nature to the detection used in known instruments. The reaction chamber and column are identified, and the location of liquid level and agglutination bands are determined via an optical system with associated detection software. The device is such that a visual inspection and detection is also possible. Figure 2 is an illustration of the expected image.

Specifically, in Figure 2, the liquid level is detected by the position of the meniscus (6), and the agglutination region is depicted by (7). The liquid level detection is essential to ensure that sufficient reagent and analyte sample were added. The strength and size of the agglutination complex is determined by its position along the length of the separation area (4) after centrifugation.

Figure 2 also illustrates another method. The agglutination complex (7) moves through the separation area at a rate dependent upon the g-forces, the size of the agglutination and the pore or opening size in the separation area. By imaging the device multiple times during centrifugation, this rate can be determined by processing the image and locating the agglutination complex in relation to the starting point and length of the separation area. I n addition, the final position is also determined.

The optical imaging can be performed either by a 'staring' system, or by a scanning system as described above.

Figure 3 shows an embodiment of the apparatus on a carrier. In this case, the carrier holds blocks of 6 apparatus. Figure 4 depicts a second method of mounting the apparatus for assay performance. In this case, a CD format is used.

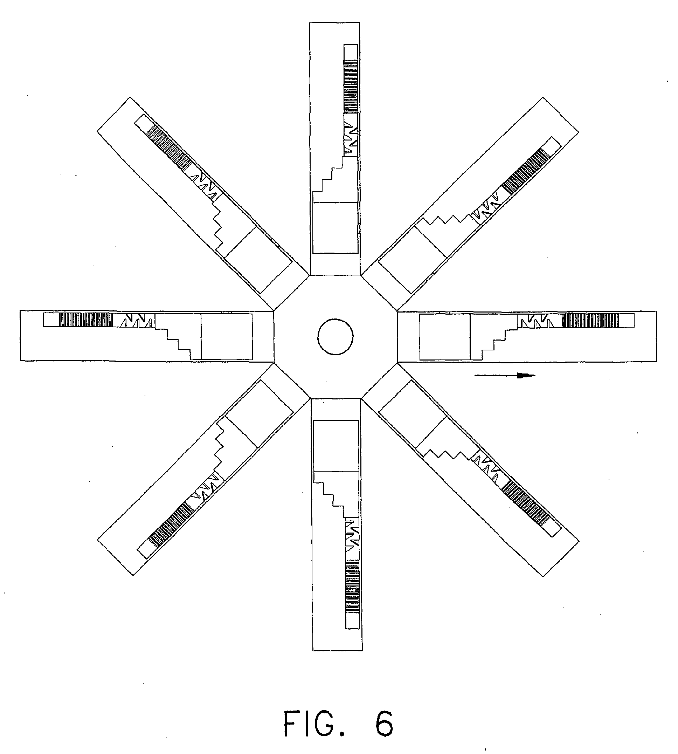

The embodiment shown in Figure 6 is a variant on the embodiment shown in Figure 3. In Figure 3, the devices at the ends of the slides may have agglutination migrating to the sides of the separation area due to g forces being at an angle to the center line. The embodiment in Figure 6 is a modification that allows the g forces to be aligned along the axis of the separation region.

By mounting the apparatus in a carrier in a vertical position, the g forces are always aligned with the axis of the carrier. In this way, the agglutination does not migrate or settle to the sides of the chamber.

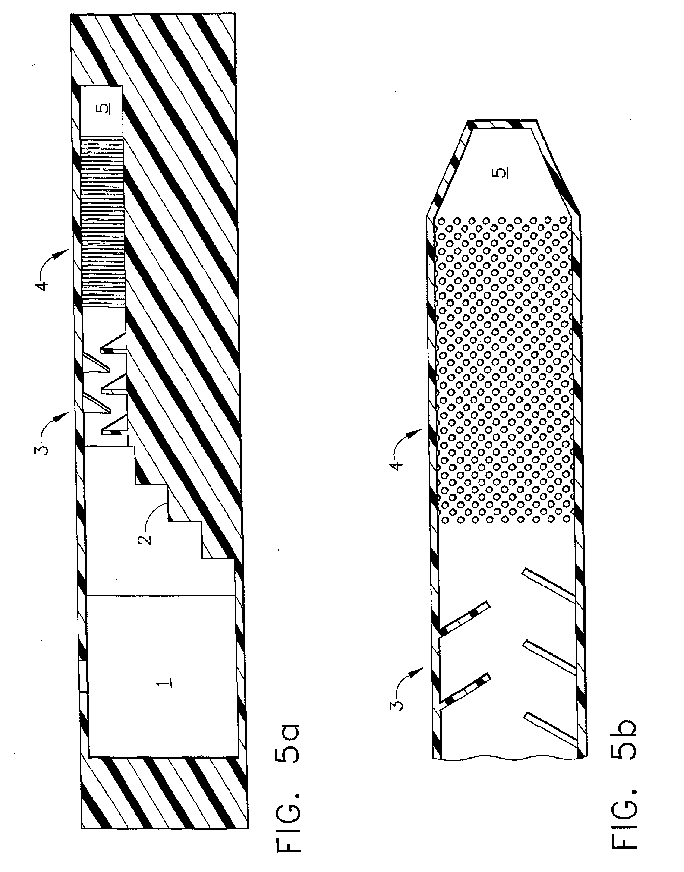

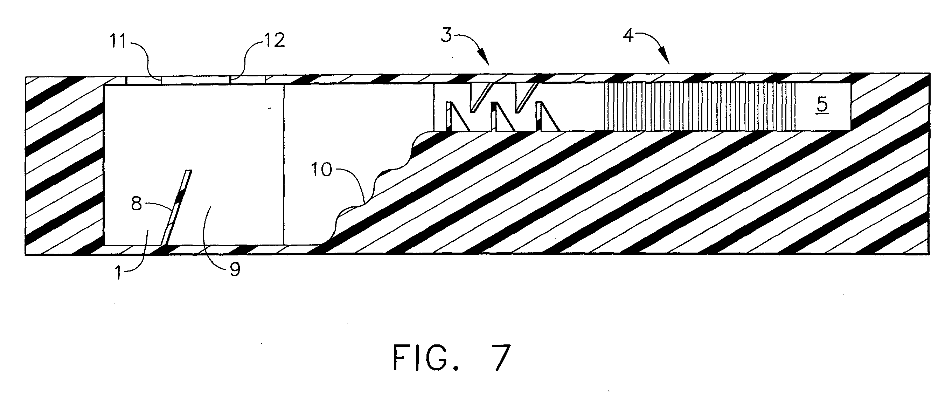

The embodiment shown in Figure 7 depicts a variation on the basic reaction chamber design shown in Figure 5. The reaction chamber is labeled (1). The secondary reaction chamber (9) may be used for additional reagent, or a prewetting agent for the separation column. Analyte and reagent are added through the inlets (11) and (12). The steps (10) are rounded versions of (2) noted in Figure 3. The slalom or enhancement section (3), separation section (4), and separation chamber end (5) are the same as in Figure 3. The rounded steps (10) and the slalom or enhancement section (3) serve to increase the mixing efficiency as the fluid is moved through to the separation area. The reaction chamber flap (baffle) (8) serves to keep fluids separate until a spin motion causes the fluid to pass from (1) to (9) due to g forces.

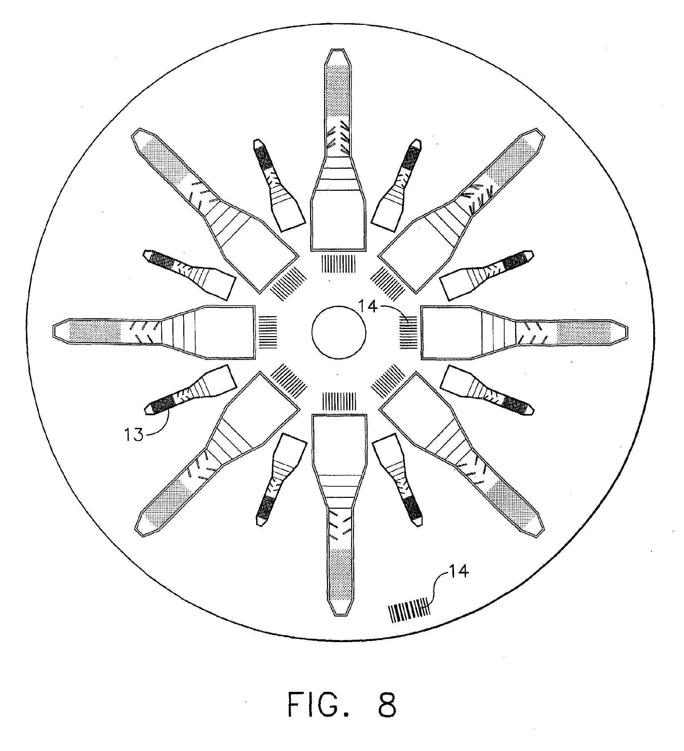

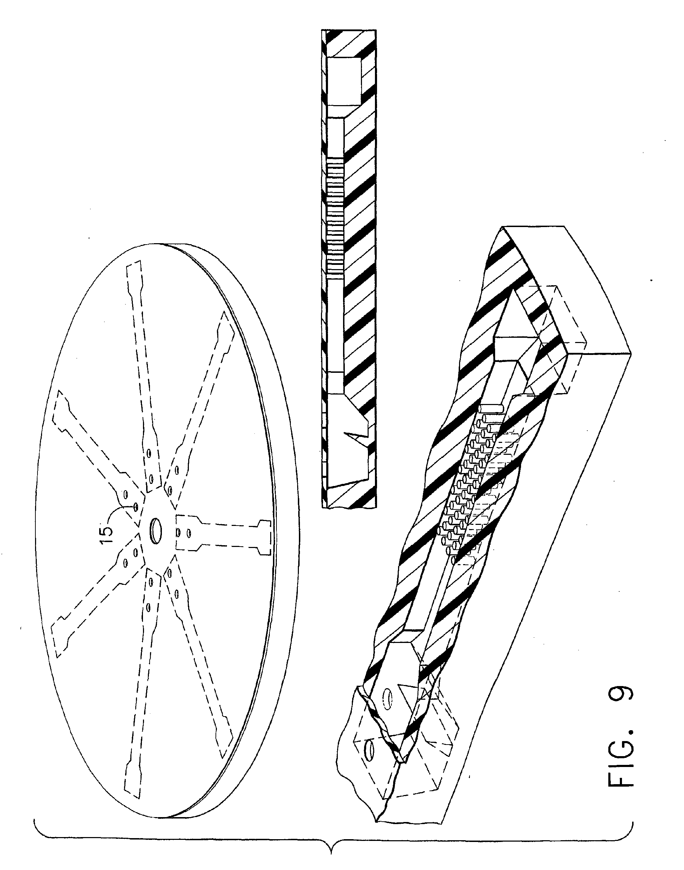

Figure 8 depicts a compact disk ("CD") such as a CD-ROM format with the addition of balancing wells (13). These wells allow the disk to be utilized in the event that not all sample wells are in use. The CD-ROM format allows for positive identification of sample, well and disk via barcodes (14) imprinted, or engraved, or stamped, or etched into the surface of the device. In addition, the use of g forces to move the agglutination through the separation area (4) allows for the scanning of the reaction in real time (Figure 9).

Figure 9 shows a perspective view of another embodiment of the apparatus and carrier according to the present invention. In the embodiment shown in Figure 9, the reaction chamber is divided with a baffle (8) to separate the fluid and reagents. Also shown is the opening (15) in the top of the CD-ROM for loading the fluid and reagents.

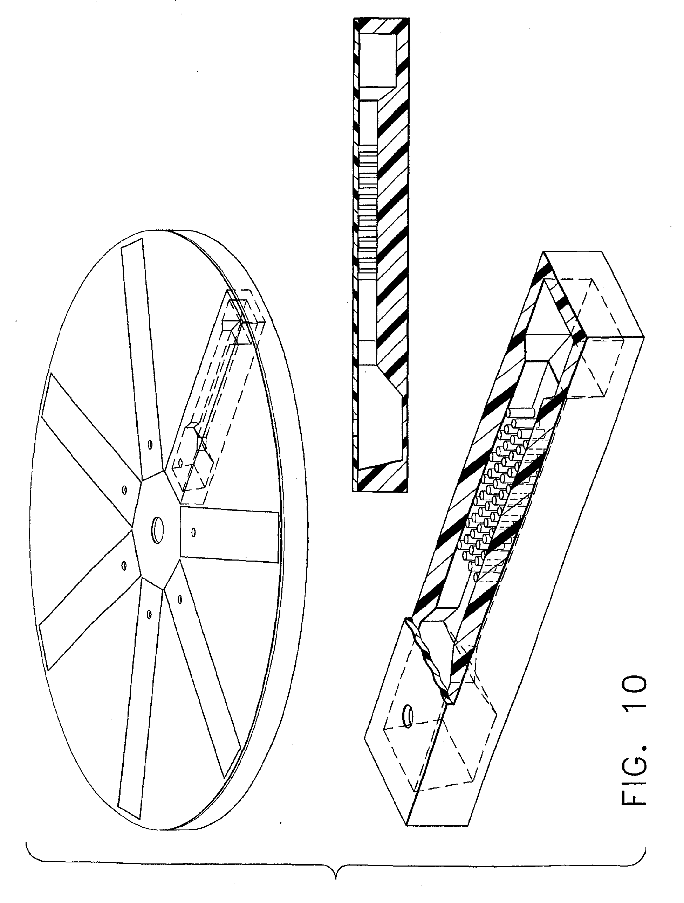

Figure 10 shows a perspective view of another embodiment of the apparatus and carrier according to the present invention. The embodiment is similar to that shown in Figure 9 except that a baffle is not shown in the reaction chamber. Also in the embodiment shown in Figure 10, the apparatus are in the form of capped inserts that are installed in the CD carrier at the time of use.

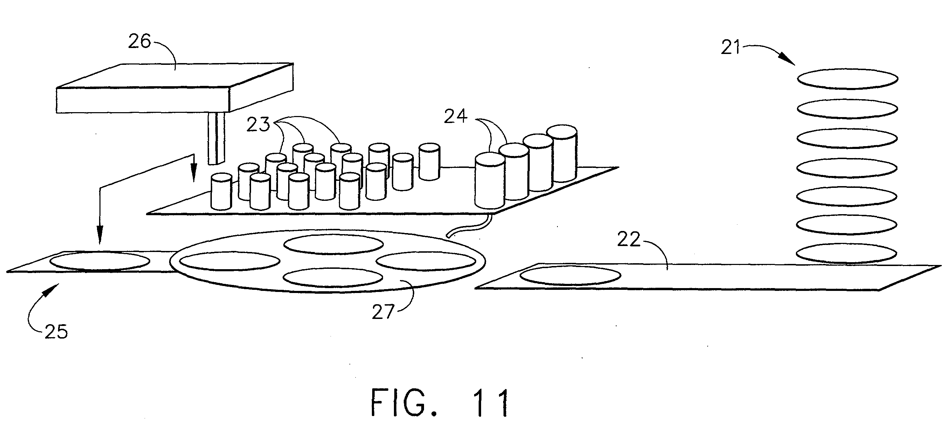

Figure 11 shows a system that includes the apparatus according to the present invention. In the embodiment shown in Figure 11, the apparatus is in a CD carrier. The CDs are loaded into a CD stack 21. A CD loader 22 transports the CD to a sample pipetting position 25, where a sample pipetting arm 26 loads sample 23 and reagent 24 into the apparatus. The CD is then loaded onto a "CD-changer" 27, which in this embodiment, contains an incubator, centrifuge and detector. As illustrated in the embodiment shown in Figure 11, the system may be supplied pre-filled with reagents. Alternatively, the reagents may be added to the system the time the assay is performed. Advantages of separate reagent addition include lower cost, improved shelf life, and reduced sensitivity to handling during shipping.

Figures 12a and 12b are top and side sectional views respectively, of a preferred embodiment according to the present invention, where the apparatus (i.e., the first, second and third sections) is a separately removable insert 31 that can be inserted onto a carrier 32, in this case an optical disk.

In a preferred method where the analyte is serum or plasma, and the reagent is red blood cell agglutinate, the relative centrifugal force (rcf) would be sufficient to move the red cells in suspension from the first section through the second section containing the elements to the third section. The speed of centrifugation is limited to prevent breakage or damage to the red blood cells. In one embodiment, a centrifuge with a variable speed motor can be employed, such as a Sero-fuge II made by Clay Adams. For example, the spin could be 900 - 1000 g for 15 to 30 seconds and 500-600 g for 30 to 45 seconds, with a final spin of 900-1000 g for 45 to 60 seconds. In another embodiment, a slower constant spin could be employed, such as 100 g for 10 minutes.

It will be apparent to those skilled in the art that various modifications and variations can be made to the compounds, compositions and processes of this invention. Thus, it is intended that the present invention cover such modifications and variations, provided they come within the scope of the appended claims and their equivalents.

The disclosure of all publications cited above are expressly incorporated herein by reference in their entireties to the same extent as if each were incorporated by reference individually.

## Claims

1. An apparatus for conducting an assay having an agglutination or size separation step, comprising:
a first section disposed to receive a fluid to be assayed; and
a second section disposed to receive the fluid from the first section upon application of a motive force to the fluid, the second section comprising elements fixed to a substrate and adapted to mix the fluid and trap agglutinated particles.

2. An apparatus according to claim 1, further comprising a third section disposed to receive fluid from the second section.

3. An apparatus according to claim 1, further wherein the assay requires an agglutination step.

4. An apparatus according to claim 1, wherein the elements are shaped as pillars.

5. An apparatus according to claim 4, wherein the cross-section of the pillars are round or oval shaped.

6. An apparatus according to claim 4, wherein the cross-section of the pillars are diamond-shaped or triangular-shaped with a point of the diamond or triangle facing an upstream direction.

7. An apparatus according to claim 4, wherein the diameter of each pillar is substantially constant in a direction along the length of the pillar.

8. An apparatus according to claim 4, wherein the pillar is conical.

9. An apparatus according to claim 1, wherein the substrate comprises a housing for the elements.

10. An apparatus according to claim 2, wherein the apparatus has a planar shape and wherein the first, second and third sections are arranged as channels in the apparatus.

11. An apparatus according to claim 10, wherein the apparatus comprises a slide or a rotatable disc.

12. An apparatus according to claim 1, wherein the width parallel to the direction of fluid flow of the first section is greater than the second section.

13. An apparatus according to claim 2, wherein the elements are spaced closer together in a direction leading away from the first section toward the third section.

14. An apparatus according to claim 1, further comprising an enhancement section disposed between the first and second section and adapted to mix the fluid.

15. An apparatus according to claim 1, further comprising a static valve that separates the first from the second section and is adapted to allow passage of fluid only upon the application of the motive force.

16. An apparatus according to claim 15, wherein the static valve comprises a series of baffles or a narrow region that retains the fluid by surface tension.

17. An apparatus according to claim 16, wherein the first section comprises a chamber having a bottom surface and the second section has a bottom surface that is higher than the first section bottom surface.

18. An apparatus according to claim 17, wherein the static valve comprises steps disposed between the first and second section and joining the first and second section bottom surface.

19. An apparatus according to claim 1, wherein the first section further comprises a first subsection for containing the fluid to be assayed and a second subsection for containing a second fluid.

20. An apparatus according to claim 1, wherein the first and second subsection are separated by a baffle disposed to allow the fluid to be assayed and the second fluid to be combined upon the application of the motive force.

21. An apparatus according to claim 1, further comprising an enhancement region disposed between the first and second section, wherein the enhancement region comprises projections extending into the path of fluid flow to cause particles to increase their proximity to enhance the strength of an agglutination reaction.

22. An apparatus according to claim 1, further comprising means to apply the motive force, wherein the motive force is one or more of an electric field, a magnetic field, a hydrodynamic force, a hydrostatic force, a gravitational force, a centrifugal force, an optical force and a thermal force.

23. An apparatus according to claim 1, further comprising a carrier for holding the first and second sections which are in the form of an insert.

24. An apparatus according to claim 2, wherein the apparatus is in the form of an disk having a central axis, and wherein the first, second and third section are arranged in the disk as channels in a direction away from the central axis, respectively.

25. An apparatus according to claim 24, wherein the apparatus further comprises a plurality of the first, second and third sections and a carrier for holding the plurality of first, second and third sections.

26. An apparatus according to claim 25, wherein the apparatus further comprises a plurality of carriers, wherein the plurality of carriers are arranged around a central axis.

27. An apparatus according to claim 24, wherein the apparatus further comprises a drive for rotating the apparatus around the central axis.

28. An apparatus according to claim 26, wherein the apparatus further comprises a drive for rotating the apparatus around the central axis.

29. An apparatus according to claim 1, further comprising a detector for detecting agglutination of the fluid to be assayed.

30. An apparatus according to claim 29, wherein the detector is an optical detector.

31. An apparatus for conducting an assay having an agglutination or size separation step, comprising:
an optical disk having a central axis;
a first section disposed to receive a fluid to be assayed, said first section including a fluid entry port to provide the fluid to be assayed;
a second section disposed to receive the fluid from the first section upon application of a motive force to the fluid, the second section comprising elements fixed to a substrate and adapted to mix the fluid and trap agglutinated particles;
a third section disposed to receive fluid from the second section, wherein the first, second and third section are arranged in the disk as channels in a direction away from the central axis, respectively;
a drive for rotating the apparatus around the central axis; and an optical detector.

32. A method for assaying a fluid that has particles to be separated or agglutinated comprising:
(a) providing a fluid to be assayed into a first section of an apparatus as claimed in claim 1;
(b) applying a motive force to the fluid to move the fluid from the first section into a second section, wherein said second section comprises elements fixed to a substrate and adapted to mix the fluid and trap particles in the fluid; and
(c) measuring a property of the fluid.

33. A method for assaying blood, comprising:
(a) providing blood or into a first section of an apparatus as claimed in claim 1;
(b) providing a reagent;
(c) combining the reagent and the blood;
(d) applying a motive force to move the combined reagent and blood from the first section into a second section, wherein said second section comprises elements fixed to a substrate and adapted to mix the blood and reagent and trap agglutinated blood cells, if any; and
(e) measuring the degree of agglutination of the blood, if any.

34. A method for assaying blood as claimed in claim 33, wherein the motive force is centrifugal force.

35. A method for assaying blood as claimed in claim 34, wherein the apparatus is a rotatable disk.

36. A method for assaying blood as claimed in claim 33, wherein the reagent is one or more antibodies derived from a human or animal source.

37. A method for assaying blood as claimed in claim 33, wherein the assay is ABO grouping, Rh typing, antigen phenotyping, ABO serum grouping, antibody detection and identification, crossmatching and titration.

38. A method for agglutination blood, comprising:
(a) providing blood or into a first chamber;
(b) providing a reagent;
(c) combining the reagent and the blood;
(d) applying a motive force to move the combined reagent and blood from the first section into a second chamber, wherein said second chamber comprises elements fixed to a substrate and adapted to mix the blood and reagent and trap agglutinated blood cells.

39. A method according to claim 31 implemented by a computer program interfacing with a computer.

40. An article of manufacture comprising a computer usable medium having computer readable program code configured to conduct the process of claim 31.
